# EUROPEAN PATENT SPECIFICATION

(11) **EP 2 632 449 B1**
(45) Date of publication and mention of the grant of the patent: **24.08.2016**
(21) Application number: 11838343.9
(22) Date of filing: 25.10.2011
(51) Int. Cl.: A61K 31/137, A61P 27/06

(54) **TREATMENT OF OCULAR AND CEREBRAL ISCHEMIA**
BEHANDLUNG VON OKULARER UND ZEREBRALER ISCHÄMIE
TRAITEMENT D'UNE ISCHÉMIE OCULAIRE ET CÉRÉBRALE

(30) Priority: 25.10.2010 US 925561
(43) Date of publication of application: 04.09.2013
(73) Proprietor: Langham Ocular And Cerebral Therapies, LLC, Cockeysville, Maryland 21030 (US)
(72) Inventor: LANGHAM, Maurice E., Cockeysville, Maryland 21030 (US)
(74) Representative: Schnappauf, Georg
(86) International application number: PCT/US2011/001807
(87) International publication number: WO 2012/060860

(56) References cited:
- WO-A2-01/78709
- US-A- 4 590 210
- US-B1- 6 403 590
- WILLIS SENSENBACH ET AL: "A COMPARISON OF THE EFFECTS OF l-NOR-EPINEPHRINE, SYNTHETIC l-EPINEPHRINE, AND U.S.P. EPINEPHRINE UPON CEREBRAL BLOOD FLOW AND METABOLISM IN MAN 1", JOURNAL OF CLINICAL INVESTIGATION, vol. 32, no. 3, 1 March 1953 (1953-03-01), pages 226-232, XP055106198, ISSN: 0021-9738, DOI: 10.1172/JCI102731
- ALM A: "THE EFFECT OF TOPICAL LEVO EPINEPHRINE ON REGIONAL OCULAR BLOOD FLOW IN MONKEYS", INVESTIGATIVE OPHTHALMOLOGY AND VISUAL SCIENCE, vol. 19, no. 5, 1980, pages 487-491, XP002721387, ISSN: 0146-0404
- IADECOLA C.: 'The overlap between neurodegenerative and vascular factors in the pathogenesis of dementia' ACTA NEUROPATHOL. vol. 120, no. 3, September 2010, pages 287 - 296, XP019847033
- LANGHAM MAURICE E ET AL: "The prevention of visual loss by ocular hypotensive drugs", INVESTIGATIVE OPHTHALMOLOGY & VISUAL SCIENCE - IOVS, ASSOCIATION FOR RESEARCH IN VISION AND OPHTHALMOLOGY, US, vol. 36, no. 4, 19 May 1995 (1995-05-19), page S966, XP009181772, ISSN: 0146-0404

## Description

### Field of Invention

This invention relates to methods for reversing cerebral and ocular ischemia, including the loss or diminishing of cerebral blood flow associated with cerebral neurodegenerative diseases including Alzheimer's.

### Background of the Invention

Evidence from experimental and clinical research has confirmed that relative ischemia and disturbance of the physiological transmural pressure acting across capillary walls entwining neurons, leads to decreased visual sensitivity in the eye and memory loss in the brain. For example, in unilateral open angle glaucoma and in unilateral macular degeneration, the blood flow and visual loss are more severe in the eye with greater loss of blood flow.

The most fundamental physiological aspects of vision and mental function are their stability and sensitivity. This stability and sensitivity are dependent on blood flow and by the capacity and response of the vascular autoregulation. In the progression of ocular and cerebral diseases these reserves are diminished and eventually completely lost.

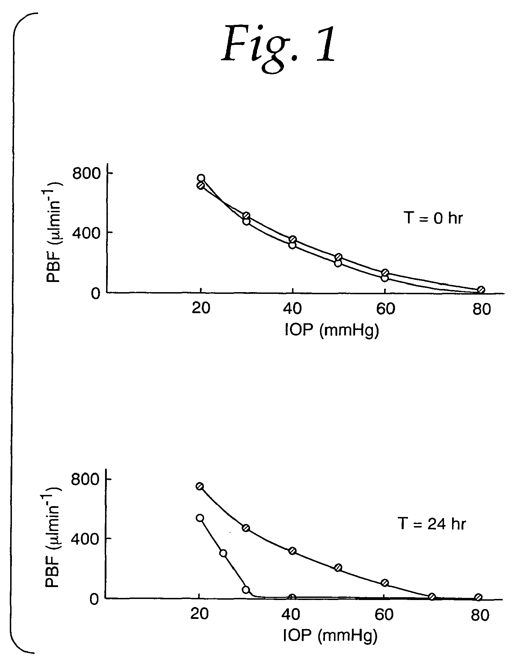

In the eye and brain the magnitude of this autoregulation is substantial. Langham, Ischemia and Loss of Vascular Autoregulation in Ocular and Cerebral Disease; A New Perspective, Springer, New York (2009). See also FIGURE 1.

The internal carotid arteries carry nearly, if not, all the blood flow to the eyes and more than half of the blood flow to the brain. The corresponding rates of blood flow are ca. 2,000 µl min⁻¹ to the eyes and 75,000 µl min⁻¹ to the brain. The internal carotid arteries are branches of the common carotid arteries and penetrate the base of the skull through the carotid canal. The ophthalmic artery arises from the anterior portion of the carotid siphon and passes with the optic nerve through the optic foramen into the orbital tissues: there it supplies blood flow to the retina, choroid and other orbital tissues.

Because the internal carotid artery ascends from the common carotid artery and the aorta, without branches, the pressure at the ophthalmic artery reflects closely the pressure in the internal carotid artery at the point it joins the common carotid artery. In turn, the maximal pressure in the carotid artery approximates closely the central cardiac pressure. The vascular resistance in the carotid arteries in healthy subjects is extremely low, resulting in the arterial pressure feeding the brain to approximate closely the central cardiac pressure.

Demands of the human brain for a relatively high blood flow and maximal oxygen tension are fulfilled by the relatively low cerebral vascular resistance compared with that of the eye. This results in a preferential flow to the brain with the arterial pressure feeding the eye being substantially less than the cerebral arterial pressure. As a consequence of this difference in perfusion pressures, abnormally high vascular resistance in the brain leads to a proportionate increase of the ophthalmic arterial pressure; *parri passu,* vascular resistances in the brain below normal, such as that induced by increased partial pressure of carbon dioxide (pCO₂), lead to a lowered ophthalmic arterial pressure.

The ophthalmic arterial pressure has been measured objectively using manometric techniques in living eyes of anesthetized animals and man. In urethane anesthetized rabbits, the femoral arterial pressure is close or equal to the central cardiac pressure, and the ophthalmic arterial pressure can be monitored from a cannula inserted in the median ear artery to a level of the ophthalmic artery. Under these conditions, increasing the intraocular pressure (IOP) in one eye from a physiological saline reservoir connected by a cannula to the anterior chamber causes the pulse amplitude to increase with increased IOP until the IOP reaches approximately 60 mmHg and then to decrease and become zero as the IOP equals the ophthalmic arterial pressure. In rabbits, the ophthalmic arterial pressure is approximately the same as the femoral arterial and the central arterial pressure. This approximate equality of the ophthalmic blood pressure and the central blood pressure in animals differs from the substantial difference in the same pressures (approximately 40 mmHg) in healthy adults.

Vascular diseases occur more frequently in older age groups and the older the individual, the more likely he or she will suffer from a chronic cerebral or visual disorder associated with the pathology of the microcirculation. In this respect, by the age of 60 years, the average blood flow through the cervical arteries of healthy adults has decreased by a mean of 30%. Numerous studies confirm the existence of abnormally low cerebral blood flow in cerebral diseases such as Alzheimer's disease (de la Torre J.C. and Stefano, Evidence that Alzheimer's Disease is a Microvascular Disorder: The Role of Constitutive Nitric Oxide, Brain Res. Rev. 2000 34(3): 119-136) and in the ocular vascular diseases open angle glaucoma, age related macular degeneration and the retinopathy of diabetes.

It is widely appreciated, based on much experimental and clinical research, that impaired vascular circulation in the eye plays a substantial role in the pathology of the retina, the optic nerve and the choroid. The closure of small vessels, the formation of microaneurysms, the growth of new vessels, hemorrhages, and the breakdown of the barriers separating the blood and the intraocular fluids are specific responses to decreased blood flow and lack of oxygen. These changes are seen in the optic nerve of patients with low and abnormally high intraocular pressure (low and high tension open angle glaucoma), in the new vessel formation close to the fovea in age related macular degeneration, in the vessel invasion of the retina of the diabetic eye and in ischemia of the cornea. (Langham, op. cit.).

Open angle glaucoma, age related macular degeneration and the retinopathy of diabetes are the major causes of blindness throughout the world. Currently, glaucoma is treated medically with drugs that reduce IOP. In this respect ocular hypotensive drugs have been approved by the U.S. Food and Drug Administration for treatment of elevated IOP in patients with ocular hypertension or open angle glaucoma.

Elevated IOP is a risk factor associated with the development of glaucoma but it is not the disease itself. The recent recognition that a large percentage of glaucoma patients never experience elevated IOP has increased the possibility that the ocular hypotensive drug has the undesirable action of decreasing ocular blood flow. This possibility has been supported by numerous studies and confirmed in the prolonged longitudinal studies on individual glaucoma patents (Table 1). With increased ocular blood flow, the vision improved whilst with a drug having equal ocular hypotensive action, but causing decreased ocular blood flow run vision worsened.

At present, there is no established treatment for macular degeneration, the retinopathy of diabetes, or for Alzheimer's disease. All these diseases are associated with a substantial reduction of ocular and/or cerebral blood flow and a complete loss of vascular autoregulation.

US 4,590,210 teaches compositions for treating ocular hypertension, but fails to mention ocular ischemia and loss of vascular ocular autoregulation.

WO 01/78709 A2 teaches the use of (-)-α-methyl norepinephrine for treating a disease associated with fibril formation or aberrant protein aggregation, but does not mention ocular or cerebral ischemia.

Sensenbach et al., J. Clin. Invest., vol. 32, pp. 226-232 (1953) and Alm, Invest. Ophthalmol. Vis. Sci., vol. 19, pp. 487-491 (1980) teach that specific catecholamine derivatives, which are not α-methyl derivatives, reduce cerebral or ocular blood flow.

Langham et al., Investigative Ophthalmology and Visual Science, vol. 36, p. S966 (1995) reports that dipivalyl-α-methyl epinephrine increases ocular blood flow, ocular pulse volume and pulsatile ocular blood flow.

It has now been found that cerebral and ocular blood flows can be enhanced and vision and memory improved by the administration to the patient of an (-) erythro isomer of an α-methyl epinephrine or α-methyl norepinephorine.

### Summary of the Invention

In a patient suffering from either abnormally low cerebral or ocular blood flow, and loss of vascular autoregulation, the blood flows are enhanced by the administration to such patient of an aqueous composition containing as the sole active ingredient an (-) erythro isomer of a catecholamine which is a member of the group consisting of α-methylepinephrine, α-methylnorepinephrine, a C₁ to C₈ aliphatic ester of α-methylepinephrine, a C₁ to C₈ aliphatic ester of α-methylnorepimephrine, and a pharmacologically acceptable acid addition salt thereof in an amount sufficient to increase the patient's cerebral or ocular blood flow by at least 5 percent by volume. The erythro (-) isomer may be administered to the patient as an ophthalmic solution in a single daily dose in the range of about 0.01 to about 2 milligrams. Other suitable routes of administration are oral, buccal, intravenous, and the like.

In a first aspect, the present invention relates to an (-) erythro isomer of a catecholamine for use in a method of treatment of diminished cerebral blood flow, wherein the (-) erythro isomer of a catecholamine is a member of the group consisting of *α*-methyl epinephrine, *α* -methyl norepinephrine, C₁ to C₈ aliphatic ester of *α*-methyl epinephrine, C₁ to C₈ aliphatic ester of *α*-methyl norepinephrine, and a pharmacologically acceptable acid addition salt thereof, and wherein the (-) erythro isomer of a catecholamine is administered to a patient in an amount sufficient to enhance cerebral blood flow.

In one embodiment of the first aspect, the (-) erythro isomer of a catecholamine is administered to the patient intravenously.

In a second aspect, the present invention relates to an aqueous composition of an (-) erythro isomer of a catecholamine for use in a method of treatment of cerebral ischemia, characteristic of the onset and progression of cerebral diseases including dementia, and specifically Alzheimer's disease, by enhancing cerebral blood flow in a patient, wherein the (-) erythro isomer is a member of the group consisting of *α*-methyl epinephrine, *α*-methyl norepinephrine, C₁ to C₈ aliphatic ester of *α*-methyl epinephrine, C₁ to C₈ aliphatic ester of *α*-methyl norepinephrine, and a pharmacological acid addition salt thereof.

In one embodiment of the second aspect, the (-) erythro isomer is administered topically to an eye of the patient in a daily dose in the range of about 0.01 to about 5 milligrams.

In one embodiment of the second aspect, the (-) erythro isomer is administered topically to an eye of the patient in a daily dose in the range of about 0.01 to about 2 milligrams.

In one embodiment of the second aspect, the (-) erythro isomer is administered to the patient orally in a daily dose in the range of about 50 to about 300 milligrams.

In a further embodiment of the second aspect, the (-) erythro isomer is administered to the patient buccally in a daily dose in the range of about 10 to about 120 milligrams.

### Brief Description of the Drawings

In the drawings,
FIG. 1 shows the effect of unilateral ligation of the common carotid on the IOP and the ocular blood flow in a conscious rabbit. The upper figure shows the rates of pulsatile blood flow in pairs of eyes prior to surgery. The lower figure shows the ocular blood flow in the eye on the operated side (open circles) and in the control eye (filled circles) 24 hours following arterial ligation. It can be seen that the ocular blood flow in the eye on the ligated side remained substantial and within the normal range, despite the severe decrease (more than 80%) in the ocular perfusion pressure.

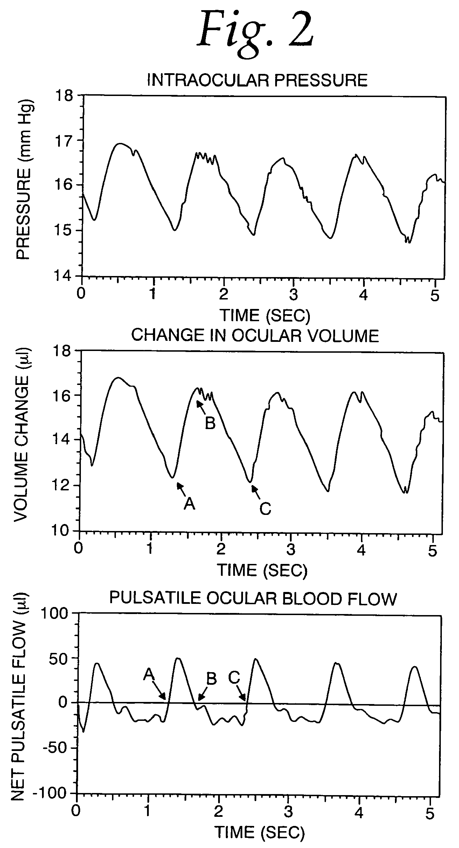

FIG. 2 shows typical intraocular pressure (IOP) recordings taken at 3 millisecond (3ms) intervals on the right eye of a healthy adult subject (age 34 years) with no history of ocular or systemic disease using the Langham digitized pneumatonometer (top graph); the middle graph shows the change in ocular volume relative to a reference ocular volume at an IOP of 10 mmHg. The bottom graph gives the net pulsatile blood flow corresponding to the above graphs. It represents an average blood flow of 825 µl min ⁻¹.

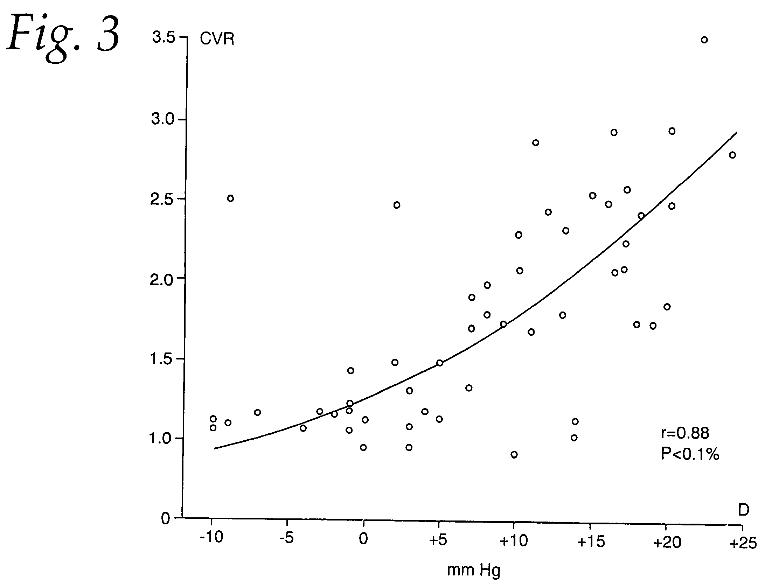

FIG. 3 shows the relationship between the cerebrovascular resistance (CVR) determined by the Kety-Schmidt method in a group of 53 patients and the deviation from the measured ophthalmic arterial pressure (Kety S.S., Physiopathology of Cerebral Circulation in Vascular Diseases of the Brain, Acta V. Congres Neurol-Internat. Lisboa 1: 343-351 (1953)).

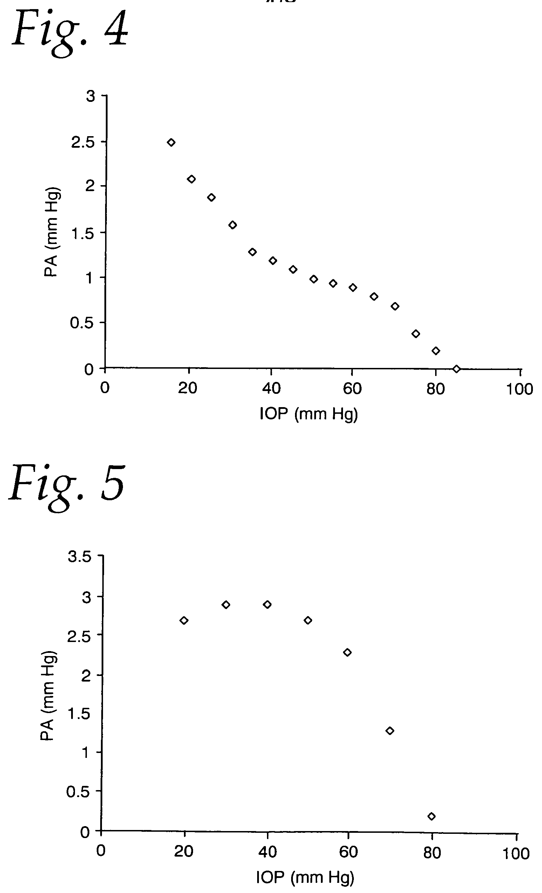

FIG. 4 shows the mean IOP/Pulse Amplitude (PA) relationship in a group of 12 healthy adult subjects. The results are expressed as the arithmetic mean ± the standard error of the mean where the standard error at each point was less than 0.1 mmHg. The PA fell to zero and vision was lost at a mean IOP of 82 mmHg (the ophthalmic arterial pressure) with the corresponding brachial arterial blood pressure of 120/85 mmHg. The S-shape of this curve reflects the change in ocular blood flow due to autoregulation.
FIG. 5 shows the IOP/PA curve assuming a linear decrease of pulsatile ocular blood flow (mean 740 µl min ⁻¹ in the undisturbed eye) to zero flow at the ophthalmic arterial pressure of approximately 85 mmHg. The PA values have been calculated using a coefficient of ocular rigidity of 0.0125, the mean of normal eyes. This relationship indicates absence of autoregulation.

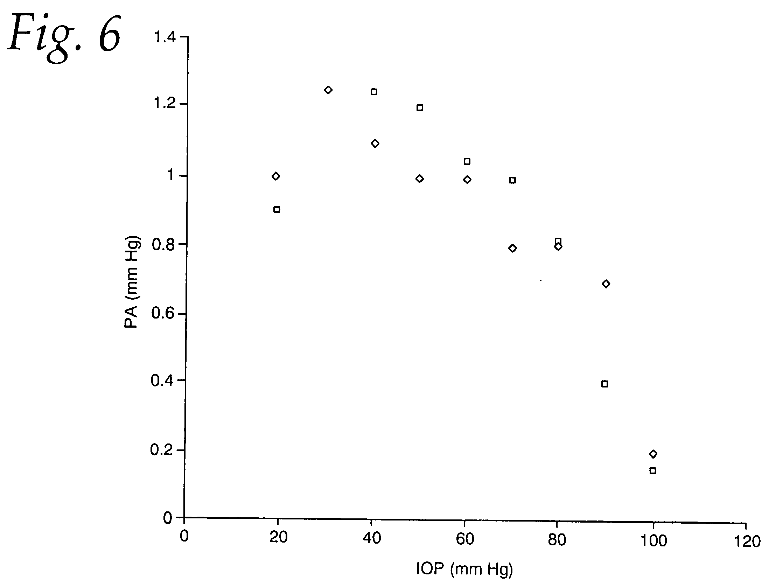

FIG. 6 shows the mean IOP/Pulse Amplitude relationship for a 65 year-old female with a diagnosis of Alzheimer's disease with visual impairment. The pulsatile blood flows were 299 and 300 µl/min in the two eyes which are substantially lower than the mean of 740 µl/min in healthy eyes. The brachial arterial blood pressure (BrAP) was 120/80 mmHg and the ophthalmic arterial pressure (OAP) ca. 102 mmHg giving OAP/BrAP ratios of 0.83 which are abnormally and statistically high (0.67 ± 0.01 is the mean in healthy subjects) and consistent with high cerebral vascular resistance.

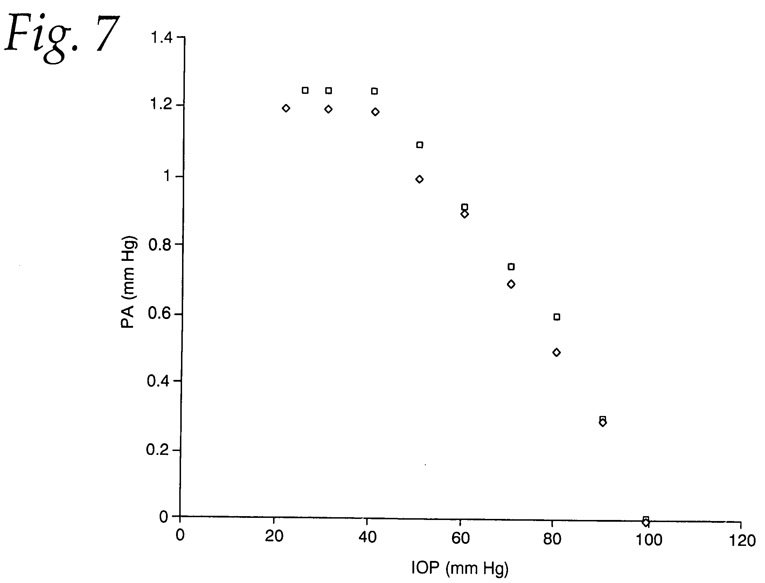

FIG. 7 shows the IOP/PA relationship in eyes of a patient with bilateral neovascular age related macular degeneration. It can be seen that the IOPs differed between pairs of eyes and the IOP/PA relationships were abnormal and consistent with the loss of autoregulation. The rates of pulsatile blood flows were 436 and 437 µl/min and well below normal.

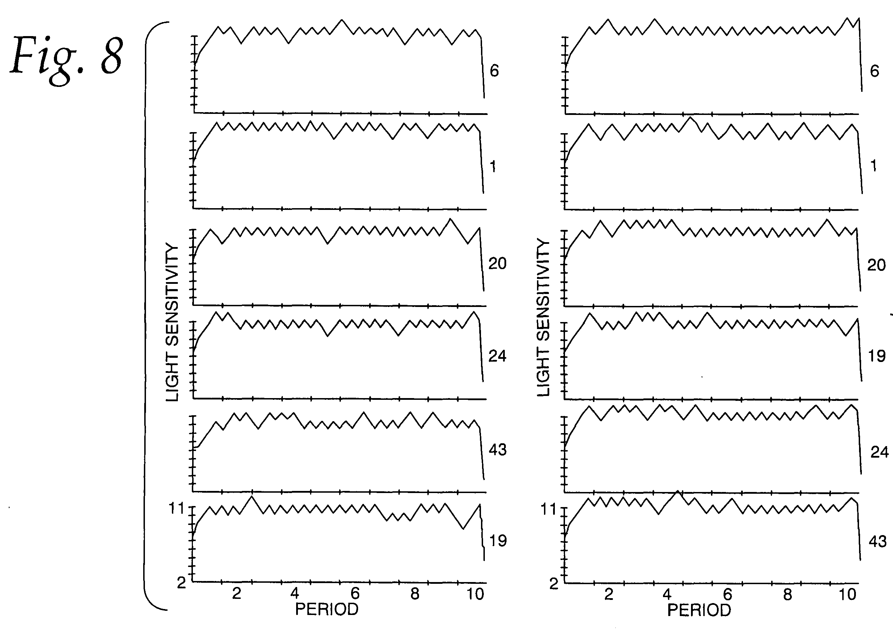

FIG. 8 shows representative examples of the time dependent differential light sensitivities in pairs of eyes of a healthy subject. The mean linear regression equations in the two eyes were y = 11.0 ± 0.05 (6) and y = 11.0 ± 0.04 (6). The ordinate gives the level of intensity. The abscissa indicates the ten test periods in minutes. The grogram was initiated at a light intensity of level 6.

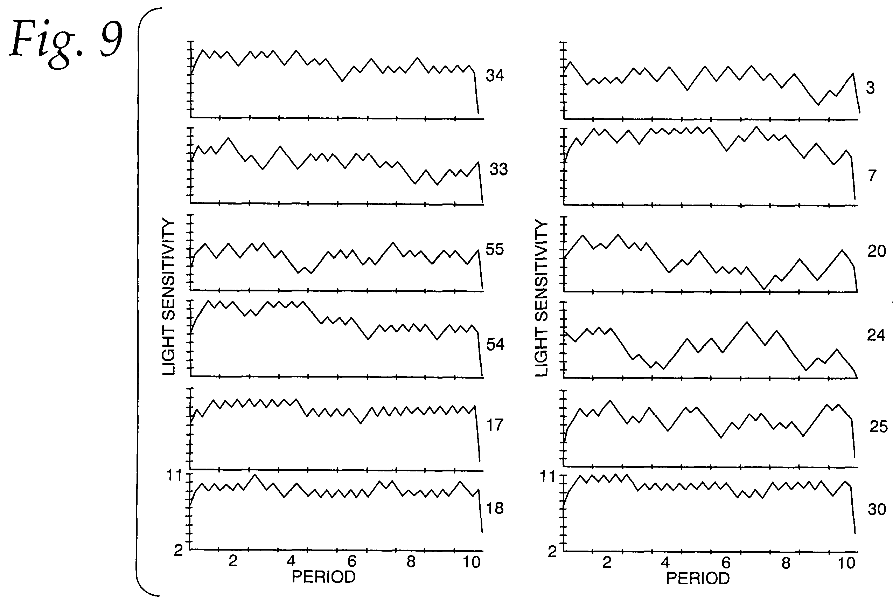

FIG. 9 shows representative recordings demonstrating the instability and loss of sensitivity of the time dependent differential visual threshold in glaucomatous eyes of two patients.

### Detailed Description of Preferred Embodiments

The compounds suitable for practicing the present invention belong to the class of compounds known as catecholamines which can be represented by the formula: 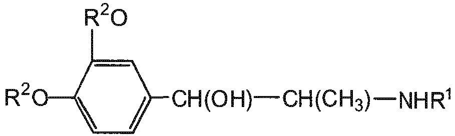 wherein R¹ can be hydrogen or methyl and R² can be hydrogen or an acyl moiety containing 1 to 8 carbon atoms, inclusive. The foregoing compounds have two asymmetric carbon atoms, thus each one of the compounds shown by the above formula can exist in four epimeric forms; i.e., as the (-) erythro, (+) erythro), (-) threo or (+) (threo) stereoisomer. For practicing the present invention, however, it has been found that only the (-) erythro stereoisomer is suitable. According to the present invention, the (-) erythro stereoisomer of a catecholamine is a member of the group consisting of *α*-methyl epinephrine, *α*-methyl norepinephrine, C₁ to C8 aliphatic ester of *α*-methyl epinephrine, C₁ to C₈ aliphatic ester of *α* -methyl norepinephrine, and a pharmacologically acceptable acid addition salt thereof. Thus, the (-) erythro stereoisomer can exist and can be used in its non-protonated or free base form as well as in its protonated or acid addition form.

Physiologically tolerable acid addition salts of the foregoing compounds can be prepared by the neutralization of the free base form with an appropriate amount of a organic or inorganic acid, examples of which are hydrochloric, hydrobromic, phosphoric, acetic, lactic, salicylic, glycolic, ascorbic, succinic, tartaric, maleic, malic, pamoic, citric, and the like. The preferred acid addition salts for the present purposes are selected from the grouping hydrochloride, ascorbate, and maleate, and are prepared from hydrochloric acid, ascorbic acid and maleic acid, respectively. Neutralization can be carried out by a variety of procedures known to the art to be generally useful for the preparation of amine acid addition salts.

The choice of the most suitable procedure in a given instance will depend on a variety of factors including convenience of operation, economic considerations, and particularly the solubility characteristics of the particular free base, the acid, and the acid addition salt. For example, if the acid is soluble in water, the free base can be dissolved in water containing an equivalent amount of the acid, and thereafter, the water can be removed by evaporation; in some instances, the salt precipitates from the aqueous solution, particularly when cooled, and evaporation is not necessary. If the acid is soluble in a relatively non-polar solvent, for example, diethyl ether or diisopropyl ether, separate solutions of the acid and free base in such a solvent can be mixed in equivalent amounts, whereupon the acid addition salt will usually precipitate because of its relatively low solubility in the non-polar solvent. Alternatively, the free base can be mixed with an equivalent amount of the acid in the presence of a solvent of moderate polarity, for example, a lower alkanol, a lower alkanone, or a lower-alkyl ester of a lower alkanoic acid. Examples of these solvents are ethanol, acetone, and ethyl acetate, respectively.

Subsequent admixture of the resulting solution of acid addition salt with a solvent of relatively low polarity, for example, diethyl ether or hexane, usually causes precipitation of the acid addition salt. The formation of acid addition salts can also be utilized for upgrading the free bases prior to formulations, if necessary. The compositions of the present invention can be administered topically to the eye in unit dosage form, as ophthalmic solutions (including physiological saline), or as ophthalmic ointments, creams, gels, or dispersions. Typical ointment bases suitable for this purpose include white petrolatum and mineral oil or liquid petrolatum. Slow release polymers or depo systems incorporating the described (-) erythro α-methyl derivatives of epinephrine and/or norepinephrine can also be employed if desired.

The term "unit dosage form" as used herein refers to physically discrete units suitable as unitary dosages for humans and animals, each unit containing a predetermined quantity of active material calculated to produce the desired therapeutic effect in association with the required diluent, i.e., carrier, or vehicle. The specifications for the novel unit dosage forms of this invention are dictated by and are directly dependent on (a) the unique characteristics of the active material and the particular therapeutic effect to be achieved, and (b) the limitations inherent in the art of compounding such active material for therapeutic use in humans as disclosed in detail in the specification. The unit dosage forms can be delivered to the eye as drops, or by suitable microdrop or spray devices typically providing a metered dose of medication, as well as orally. The amount of active ingredient that is to be administered depends on the age, weight of the patient, the particular condition to be treated, the frequency of administration, and the like.

When administered topically to the eye of a patient, the dose can range from about 0.01 to about 5 milligrams daily given as a single dose or in 3 or 4 divided doses. Preferably, the daily adult dose administered topically is about 0.01 to about 2 milligrams.

The active ingredient can be administered to the patient also orally, buccally, or intravenously. For oral administration, a preferred daily dose is in the range of about 50 to about 300 milligrams. For buccal administration a preferred daily dose is in the range of about 10 to about 125 milligrams. For intravenous administration a preferred daily dose is about 5 to about 100 milligrams.

A preferred oral dosage form is a solution or a tablet. A typical oral tablet contains 100 to 250 milligrams of the (-) erythro isomer together with inactive ingredients such as calcium disodium edetate, cellulose, citric acid, colloidal silicon dioxide, ethylcellulose, guar gum, hydroxypropyl methylcellulose, iron oxide, magnesium stearate, propylene glycol, talc, titanium dioxide, and a color additive. A typical buccal tablet contains 75 to 125 milligrams of the (-) erythro isomer in a readily erodible matrix usually constituted by hydroxypropyl methyl cellulose and ethyl cellulose. Another typical buccal tablet comprises about 125 milligrams of the (-) erythro isomer in a matrix that melts at body temperature and comprises 70-90 weight percent polyethylene glycol having a molecular weight of about 1,000 (melting point about 37°C.), up to 4 weight percent polyethylene glycol having a molecular weight of about 3,350 to about 8,000, up to 4 weight percent long chain saturated carboxylic acid, 0.1 to 4 weight percent polyethylene oxide having a molecular weight of about 100,000 to about 5,000,00 and 10 to 20 weight percent of colloidal silicone.

Yet another type of buccal tablet comprises about 100 milligrams of the (-) erythro isomer in an erodible matrix constituted by 800 milligrams lactose, 870 milligrams sucrose, 100 milligrams acacia, 60 milligrams talc, and 10 milligrams magnesium stearate.

The concentration of active ingredient in an ophthalmic solution usually is within the range of 0.001 to 0.5 weight percent by volume. This range is preferably from about 0.001 to about 0.15 weight percent by volume. Higher concentrations of solutions, as for example, those at about 0.2 to about 0.5 weight percent by volume, as well as lower concentrations, can be employed (as for example, in a solution in combination with a miotic, e.g., pilocarpine, or with a sympathomimetric amine), provided that the ultimate solution concentrations of the present (-) erythro α-methyl derivatives of epinephrine or norepinephrine together with the exogenous sympathomimetic amines and the miotic are non-irritating. These compositions can be administered in unit dosage form, i.e., dropwise, one to three times daily. After the patient has responded, as determined by enhanced cerebral blood flow, the daily regimen can be reduced to once a day or once every other day or less, as a maintenance dose for continued effect. As stated hereinbefore, the concentration of active ingredient in the present compositions can be varied. It is necessary, however, that the active ingredient be present in an amount such that a suitable therapeutic dosage will be delivered to the patient. While several unit dosage forms can be administered at about the same time, administration at appropriate periodic intervals to achieve the desired effect is preferable. Activity increases with concentration of the active ingredient in the unit dose, and in general, it has been found to be desirable to maintain unit dosage concentrations below that level at which any systemic action due to the α-methyl derivative present is observable. Such concentrations generally fall within the above-described ranges; however, it is to be understood that these general ranges can be modified in certain instances to suit the needs and responses of an individual patient. Therefore, any dose which will produce the desired enhancement of cerebral or ocular blood flow without irritation, and furthermore, falls below the toxic dose, in most instances below the LD₅₀ dose of the particular α-methyl derivative present, can be employed.

For the present purposes sterile physiological saline is a suitable vehicle. Other suitable ophthalmic vehicles are well known in the art and are fully described in such standard reference works as Remington's Pharmaceutical Sciences, Martin and Cook, Mack Publishing Co., Easton, PA. 13th Edition (1965). The following is a suitable example. (The percentages in the following examples refer to a percent weight by volume).

**STERILE VEHICLE**

| **Ingredient** | **Percent w/v** |
|---|---|
| Oxine Sulfate | 0.01 |
| Sodium bisulfite | 0.3 |
| Phenylmercuric acetate | 0.002 |
| Sodium hydroxide or | |
| Hydrochloric acid to pH 3.5-6 Water, q.s. | |

In the foregoing composition, the oxine sulfate (8-hydroxy-quinolone sulfate) and the sodium bisulfite act as antioxidants and the concentration thereof can vary tenfold (the former up to about 0.1 percent, and the latter down to about 0.03 percent). In addition to these specific antioxidants, any ophthalmic antioxidant can be employed. These are more fully described in Remingon (*supra*). Phenyl mercuric acetate is employed as a preservative. Any preservative suitable for ophthalmic formulation such as those described in Remington *(supra*) can be employed. A pH value range from about 3.5-8 can be utilized, although a pH value within the physiological range is preferred. When employing a buffered system, it is preferred to utilize a pH value of about 6.0 to about 8. With a buffered system, pH value can be adjusted by adjusting the concentration and, thereupon, altering the ratio of the buffered tonicity so as to maintain an isotonic solution. Although buffers can be used at varying pH values, when the pH value is less than 6, sodium hydroxide or hydrochloric acid can be employed for adjustment. When using a buffered system, it is preferred to adjust the pH range to that of the physiological pH value range of about 6 to 7.5,or 8. U.S. Patent No. 3,149,035 to Riegelman sets forth additional specific suitable sterile vehicles than can be employed in formulating the compositions of this invention.

The pH value of the foregoing, specific sterile vehicle can be adjusted using base or acid. Also, standard buffering agents such as those described in Remington (*supra*) or in the Merck Index, 9th Ed., page Misc. 97, (1976), can be used so long as these buffering agents are suitable for an ophthalmic formulation.

Typical formulations effective for practicing the present invention are set forth hereinbelow:

| **Ingredient** | **Percent w**/**v** |
|---|---|
| **FORMULATION 1** | |
| (-) erytho-α-methyl epinephrine | 0.1 |
| Oxine Sulfate | 0.01 |
| Sodium bisulfite | 0.3 |
| Boric acid | 0.8 |
| Sodium borate | 0.6 |
| Phenylmercuric acetate Water, q.s. | 0.002 |

| **Ingredient** | **Percent w/v** |
|---|---|
| **FORMULATION I1** | |
| (-) erytho-α-methyl norepinephrine | 0.25 |
| Oxine Sulfate | 0.01 |
| Sodium bisulfite | 0.3 |
| Boric acid | 0.8 |
| Sodium borate | 0.6 |
| Phenylmercuric acetate | 0.002 |
| Water, q.s. | |

As mentioned hereinbefore, the compositions of this invention can also be formulated and administered as ophthalmic ointments compounded, for example, by mixing finely milled powdered ingredients with a small amount of white petrolatum and livigating or otherwise combining until a uniform distribution is achieved. The balance of white petrolatum is added by geometric addition until the desired dosage form is achieved.

The (-) erythro isomer of a catecholamine according to the present invention has been tested by standard laboratory procedures and found to possess the capability of increasing ocular and cerebral blood flows. The ability to improve cerebral blood flow in patients increases the therapeutic potential to treat ischemic diseases of the brain, including Alzheimer's disease.
***1. The Intraocular pressure and pulse form***. The Langham pneumatic digitized tonometer was used to make continuous recordings of the intraocular pressure and its pulsatile character. This recording system provides the steady state IOP and the form of the IOP pulse with an accuracy equal to a manometric recording. The pulsatile ocular blood flow is evaluated from the amplitude of the individual pulses using the known pressure/volume relation of living human eyes. Langham, M.E., Farrell, R.A., O'Brien V., Silver, D.M., Non-Invasive Measurement of Pulsatile Ocular Blood Flow in the Human Eye (Eds., Lambrou G.N., Greve E.) pp. 93-99; Kugler and Ghedini, Amsterdam (1989).
***2. The IOP*/*PA Relation, Autoregulation and Ophthalmic Arterial Pressure.*** The IOP/PA was measured using a scleral suction cup applied to the perilimbal area of the cornea and applying suction to the cup to raise the IOP in discrete steps. At each step the IOP was recorded using the Langham, digitized computer tonometer; the mean relation for healthy eyes is shown in FIG. 4. The S-shape of this curve in healthy eyes reflects the autoregulation. In eyes that have lost autoregulation the PBF decreases linearly with IOP and the curve is hyperbolic in shape as shown in FIG. 5. The IOP at which the pulse becomes zero is the ophthalmic arterial pressure.
***3. Non*-*Invasive Evaluation of Cerebral Blood Flow.*** The blood flow to the brain was evaluated from the level of the ophthalmic arterial pressure compared to the mean ophthalmic arterial pressure in healthy eyes. The mean ophthalmic arterial systolic pressure is 82 mmHg and about 40 mmHg less than the pressure of 120 mmHg feeding the brain. The ophthalmic arterial pressure is modulated by the cerebral vascular resistance and with increased flow resistance the ophthalmic arterial pressure increases proportionately. For example, in patients with Alzheimer's disease the ophthalmic arterial pressure has been found to be approximately 20 mmHg higher than normal, indicating an increase of about 50% in the cerebro-vascular resistance. A typical IOP/PA relation in a patient diagnosed with Alzheimer's disease is shown in FIG. 5. It will be noted that the ophthalmic arterial pressures in both eyes are ca. 100 mmHg which is abnormally high compared to the mean of 82 mmHg in healthy individuals. These values imply an increased mean cerebral vascular resistance of 34% and an absence of cerebral autoregulation.

**Table 1. Summary of the IOP, the pulsatile blood flow, the visual performance, the relative scotomas and the stability of the differential light sensitivity in five glaucoma patients prior to and during three years of treatment with topical applications of timolol (0.25-0.50% b.i.d.), followed by three years treatment with applications of 0.25% clonidine (b.i.d.). Results in pairs of eyes did not differ significantly and the mean in pair of eyes were used in the statistical analysis (*p ≤ 0.01).**

| **Parameter** | **Prior** | **Timolol** | **Tim-Prior** | **Clonidine** | **Clon-Timolol** |
|---|---|---|---|---|---|
| IOP (mmHg) | 24 ± 0.5 | 20.8 ± 0.6 | -3.1 ± 0.6* | 20.0 ± 1.0 | -0.8 ± 0.5 |
| PBF (µl min⁻¹) | 541 ± 24 | 365 ± 20 | -177 ± 33* | 557 ± 30 | 192 ±71 |
| Vis. P. | 652 ± 22 | 533 ± 20 | -116 ± 2* | 564 ± 30 | 31 ± 9 |
| Rel. Scot | 1 | 3 | -- | 3 | -- |
| Stability | 5 decreased | 5 decreased | 5 decreased | 5 improved | 5 improved |
| Years | 1 and 2 | 3, 4, and 5 | | 6, 7 and 8 | |

**Table 2. The time course of the IOP and the ocular pulsatile blood flow (PBF) in six conscious rabbits to a single topical application of a 0.025% aqueous solution of the (-) erythro isomer of DPαMeEp (pH 7.40).**

| **Intraocular Pressure (IOP), mmHg** | | | |
|---|---|---|---|
| **Time (hr)** | **Control** | **Treated** | **Difference** |
| 0 | 19.5 ± 0.8 | 19.4 ± 0.7 | -0.1 ± 0.1 |
| 1 | 19.4 ± 0.7 | 13.0 ± 0.5 | -6.4 ± 0.3 |
| 3 | 19.5 ± 0.7 | 12.8 ± 0.4 | -6.5 ± 0.4 |
| 5 | 19.1 ± 0.5 | 13.8 ± 0.4 | -5.3 ± 0.4 |
| 12 | 19.5 ± 0.6 | 15.6 ± 0.6 | -3.9 ± 0.3 |
| 24 | 19.6 ± 0.7 | 19.7 ± 0.7 | 0 ± 0 |

| **Pulsatile Blood flow µl/min** | | | |
|---|---|---|---|
| 0 | 647 ± 27 | 651 ± 28 | 3 ± 3 |
| 1 | 624 ± 30 | 880 ± 32 | 255 ± 24 |
| 3 | 634 ± 30 | 957 ± 33 | 332 ± 29 |
| 5 | 633 ± 30 | 963 ± 30 | 328 ± 24 |
| 12 | 634 ± 31 | 924 ± 29 | 290 ± 31 |
| 24 | 672 ± 38 | 682 ± 41 | 10 ± 4 |

**Table 3: The steady state IOP and the pulsatile blood flow in five healthy human subjects given topical unilateral applications of 30 µl of a 0.025% solution of the (-) erythro isomer of dipivalyl α MeEp (DPαMe EP) at 0, 12, 24, 36, 48, 60, 72, 84 and 96 hr. The mean ophthalmic pressure prior to the initiation of the study was 82 ± 2 (5) mmHg and on the third day of the study was 70 ± 12 (5) mmHg. This reflects a significant decrease of the cerebral vascular resistance.**

| **Intraocular Pressure (IOP), mmHg** | | | |
|---|---|---|---|
| **Time (hr)** | **Control** | **Treated** | **Difference** |
| 0 | 15.5 ± 0.5 | 15.5 ± 0.5 | 0 ± 0.1 |
| 2 | 15.4 ± 0.5 | 12.0 ± 0.7 | -3.4 ± 3.4 |
| 6 | 15.8 ± 0.4 | 13.2 ± 9.2 | -2.6 ± 2.6 |
| 12 | 15.8 ± 0.6 | 14.0 ± 0.4 | -1.8 ± 1.1 |
| 24 | 15.6 ± 0.6 | 15.0 ± 0.4 | -0.6 ± 0.3 |
| 28 | 16.3 ± 0.3 | 12.4 ± 0.5 | 2.9 ± 0.4 |
| 52 | 15.5 ± 0.2 | 12.8 ± 0.4 | -2.7 ± 0.6 |
| 78 | 15.5 ± 0.3 | 11.8 ± 0.5 | -3.7 ± 0.4 |
| 100 | 16.0 ± 0.4 | 12.7 ± 0.4 | 3.3 ± 0.4 |

| **Pulsatile Blood flow µl/min** | | | |
|---|---|---|---|
| 0 | 679 ± 34 | 675 ± 35 | 4 ± 6 |
| 2 | 681 ± 31 | 818 ± 30 | 127 ± 27 |
| 6 | 571 ± 35 | 802 ± 17 | 131 ± 14 |
| 12 | 676 ± 32 | 780 ± 15 | 104 ± 12 |
| 24 | 676 ± 34 | 758 ± 30 | 82 ± 7 |
| 28 | 676 ± 23 | 790 ± 34 | 132 ± 19 |
| 52 | 674 ± 21 | 764 ± 14 | 110 ± 24 |
| 78 | 676 ± 23 | 832 ± 24 | 156 ± 8 |
| 100 | 674 ± 29 | 812 ± 15 | 138 ± 8.5 |

**Table 4: The IOP and PBF responses in four glaucomatous monkeys to a topical single unilateral application of 0.05 mg of the (-) erythro isomer of DPαMeEp at time 0 hr. The ocular pulsatile blood flow increased in all the treated glaucomatous eyes by a mean of 135 ± 8 µl/min. The decrease of the IOP lasted for more than 36 hr. in all treated glaucomatous eyes.**

| **Intraocular Pressure (IOP), mmHg** | | |
|---|---|---|
| **Time (hr)** | **Control** | **Treated** |
| 0 | 20.4 ± 1.23 | 33.4 ± 1.94 |
| 1 | 21.7 ± 1.08 | 26.3 ± 3.17 |
| 3 | 21.3 ± 0.97 | 21.7 ± 3.77 |
| 8 | 21.0 ± 1.35 | 21.9 ± 3.31 |
| 10 | 21.1 ± 1.08 | 22.1 ± 2.76 |
| 12 | 20.0 ± 0.95 | 21.8 ± 2.61 |
| 24 | 19.7 ± 0.97 | 24.6 ± 2.37 |
| 26 | 20.6 ± 0.84 | 26.3 ± 2.16 |
| 28 | 21.0 ± 1.27 | 25.0 ± 2.09 |
| 30 | 19.7 ± 1.38 | 25.0 ± 2.28 |
| 36 | 21.4 ± 0.43 | 27.3 ± 2.09 |

**Table 5: Lack of response of the IOP in four glaucomatous monkeys to a topical single unilateral application of 0.05 mg of the (+) erythro isomer of DPαMeEp at time 0 hr. Neither the IOP nor the pulsatile ocular blood flow in the control and treated eyes of individual monkeys changed significantly over the experimental period of 24 hr.**

| **Intraocular Pressure (IOP), mmHg** | | |
|---|---|---|
| **Time (hr)** | **Control** | **Treated** |
| 0 | 20.4 ± 1.23 | 36.8 ± 1.94 |
| 1 | 21.7 ± 1.08 | 36.3 ± 3.17 |
| 3 | 21.3 ± 0.97 | 35.0 ± 2.28 |
| 6 | 21.0 ± 1.35 | 36.8 ± 3.31 |
| 24 | 21.1 ± 1.08 | 34.6 ± 2.76 |

| **Pulsatile Blood flow µl/min** | | |
|---|---|---|
| 0 | 714 ± 10 | 564 ± 16 |
| 1 | 712 ± 10 | 555 ± 26 |
| 3 | 689 ± 14 | 575 ± 22 |
| 6 | 713 ± 12 | 564 ± 23 |
| 24 | 701 ± 7 | 549 ± 27 |

The foregoing specification and the data are illustrative. The scope of the invention is defined by the claims. Variants within the scope of the invention are possible.

## Claims

1. An (-) erythro isomer of a catecholamine for use in a method of treatment of diminished cerebral blood flow, wherein the (-) erythro isomer of a catecholamine is a member of the group consisting of α-methyl epinephrine, α-methyl norepinephrine, C₁ to C₈ aliphatic ester of α-methyl epinephrine, C₁ to C₈ aliphatic ester of α-methyl norepinephrine, and a pharmacologically acceptable acid addition salt thereof, and wherein the (-) erythro isomer of a catecholamine is administered to a patient in an amount sufficient to enhance cerebral blood flow.

2. An aqueous composition of an (-) erythro isomer of a catecholamine for use in a method of treatment of cerebral ischemia, characteristic of the onset and progression of cerebral diseases including dementia, and specifically Alzheimer's disease, by enhancing cerebral blood flow in a patient, wherein the (-) erythro isomer is a member of the group consisting of α-methyl epinephrine, α-methyl norepinephrine, C₁ to C₈ aliphatic ester of α-methyl epinephrine, C₁ to C₈ aliphatic ester of α-methyl norepinephrine, and a pharmacological acid addition salt thereof.

3. The aqueous composition for use in accordance with claim 2, wherein the (-) erythro isomer is administered topically to an eye of the patient in a daily dose in the range of about 0.01 to about 5 milligrams.

4. The aqueous composition for use in accordance with claim 2, wherein the (-) erythro isomer is administered topically to an eye of the patient in a daily dose in the range of about 0.01 to about 2 milligrams.

5. The aqueous composition for use in accordance with claim 2, wherein the (-) erythro isomer is administered to the patient orally in a daily dose in the range of about 50 to about 300 milligrams.

6. The aqueous composition for use in accordance with claim 2, wherein the (-) erythro isomer is administered to the patient buccally in a daily dose in the range of about 10 to about 120 milligrams.

7. The (-) erythro isomer of a catecholamine for use in accordance with claim 1, wherein the (-) erythro isomer is administered to the patient intravenously.

## Patentansprüche

1. (-)-Erythro-Isomer eines Catecholamins zur Verwendung in einem Verfahren zur Behandlung von verringertem cerebralem Blutfluss, wobei das (-)-erythro-Isomer eines Catecholamins ein Mitglied der Gruppe ist, die aus α-Methylepinephrin, α-Methylnorepinephrin, C₁-C₈-aliphatischem Ester von α-Methylepinephrin, C₁-C₈-aliphatischem Ester von α-Methylnorepinephrin und einem pharmakologisch verträglichen Säureadditionssalz davon besteht, und wobei das (-)-erythro-Isomer eines Catecholamins an einen Patienten in einer Menge verabreicht wird, die ausreichend ist, um cerebralen Blutfluss zu verstärken.

2. Wässrige Zusammensetzung eines (-)-erythro-Isomers eines Catecholamins zur Verwendung in einem Verfahren zur Behandlung von cerebraler Ischämie, die für den Ausbruch und den Verlauf von cerebralen Erkrankungen, einschließlich Demenz und insbesondere Alzheimer-Krankheit charakteristisch ist, durch Verstärken von cerebralem Blutfluss in einem Patienten, wobei das (-)-erythro-Isomer ein Mitglied der Gruppe ist, die aus α-Methylepinephrin, α-Methylnorepinephrin, C₁-C₈-aliphatischem Ester von α-Methylepinephrin, C₁-C₈-aliphatischem Ester von α-Methylnorepinephrin und einem pharmakologisch verträglichen Säureadditionssalz davon besteht.

3. Wässrige Zusammensetzung zur Verwendung nach Anspruch 2, wobei das (-)-erythro-Isomer topisch an ein Auge des Patienten in einer täglichen Dosis im Bereich von etwa 0,01 bis etwa 5 mg verabreicht wird.

4. Wässrige Zusammensetzung zur Verwendung nach Anspruch 2, wobei das (-)-erythro-Isomer topisch an ein Auge des Patienten in einer täglichen Dosis im Bereich von etwa 0,01 bis etwa 2 mg verabreicht wird.

5. Wässrige Zusammensetzung zur Verwendung nach Anspruch 2, wobei das (-)-erythro-Isomer oral an den Patienten in einer täglichen Dosis im Bereich von etwa 50 bis etwa 300 mg verabreicht wird.

6. Wässrige Zusammensetzung zur Verwendung nach Anspruch 2, wobei das (-)-erythro-Isomer bukkal an den Patienten in einer täglichen Dosis im Bereich von etwa 10 bis etwa 120 mg verabreicht wird.

7. (-)-Erythro-Isomer eines Catecholamins zur Verwendung nach Anspruch 1, wobei das (-)-erythro-Isomer intravenös an den Patienten verabreicht wird.

## Revendications

1. Isomère (-) érythro d'une catécholamine pour utilisation dans un procédé de traitement d'un débit sanguin cérébral diminué, dans lequel l'isomère (-) érythro d'une catécholamine est un membre du groupe consistant en α-méthyl épinéphrine, α-méthyl norépinéphrine, ester aliphatique en C₁ à C₈ d'α-méthyl épinéphrine, ester aliphatique en C₁ à C₈ d'α-méthyl norépinéphrine, et un sel d'addition de celui-ci à un acide pharmacologiquement acceptable, et dans lequel l'isomère (-) érythro d'une catécholamine est administré à un patient en une quantité suffisante pour améliorer le débit sanguin cérébral.

2. Composition aqueuse d'un isomère (-) érythro d'une catécholamine pour utilisation dans un procédé pour le traitement d'ischémie cérébrale, caractéristique de l'apparition et de la progression de troubles cérébraux incluant la démence, et en particulier la maladie d'Alzheimer, par amélioration du débit sanguin cérébral chez un patient, tandis que l'isomère (-) érythro est un membre du groupe consistant en α-méthyl épinéphrine, α-méthyl norépinéphrine, ester aliphatique en C₁ à C₈ d'α-méthyl épinéphrine, ester aliphatique en C₁ à C₈ d'α-méthyl norépinéphrine, et un sel d'addition de celui-ci à un acide pharmacologique.

3. Composition aqueuse pour utilisation selon la revendication 2, dans laquelle l'isomère (-) érythro est administré par voie topique à un oeil du patient à une dose journalière dans l'intervalle d'environ 0,01 à environ 5 milligrammes.

4. Composition aqueuse pour utilisation selon la revendication 2, dans laquelle l'isomère (-) érythro est administré par voie topique à un oeil du patient à une dose journalière dans l'intervalle d'environ 0,01 à environ 2 milligrammes.

5. Composition aqueuse pour utilisation selon la revendication 2, dans laquelle l'isomère (-) érythro est administré au patient par voie orale à une dose journalière dans l'intervalle d'environ 50 à environ 300 milligrammes.

6. Composition aqueuse pour utilisation selon la revendication 2, dans laquelle l'isomère (-) érythro est administré au patient par voie buccale à une dose journalière dans l'intervalle d'environ 10 à environ 120 milligrammes.

7. Isomère (-) érythro d'une catécholamine pour utilisation selon la revendication 1, dans lequel l'isomère (-) érythro est administré au patient par voie intraveineuse.
